# EUROPÄISCHE PATENTSCHRIFT

(11) **EP 0 615 724 B1**
(45) Veröffentlichungstag und Bekanntmachung des Hinweises auf die Patenterteilung: **10.06.1998**
(21) Anmeldenummer: 94102366.5
(22) Anmeldetag: 17.02.1994
(51) Int. Cl.: A61B 17/04, A61B 17/28

(54) **Chirurgische Nahtklemme, insbesondere Tabaksbeutel-Nahtklemme**
Surgical suturing clamp particularly for purse string sutures
Pince de suture chirurgicale, en particulier pour suture en cordon de bourse

(30) Priorität: 17.03.1993 DE 4308454
(43) Veröffentlichungstag der Anmeldung: 21.09.1994
(73) Patentinhaber: Köckerling, Ferdinand, Dr., D-91054 Erlangen (DE); Schneider, Ignaz, Dr., 91301 Forchheim (DE)
(72) Erfinder: Köckerling, Ferdinand, Dr., D-91054 Erlangen (DE); Schneider, Ignaz, Dr., 91301 Forchheim (DE)
(74) Vertreter: Rau, Manfred, Dr. Dipl.-Ing.

(56) Entgegenhaltungen:
- EP-A- 0 512 725
- DE-A- 4 127 812
- DE-U- 9 213 263
- GB-A- 2 081 099

## Beschreibung

Die Erfindung betrifft eine chirurgische Nahtklemme, insbesondere eine Tabaksbeutel-Nahtklemme zum Legen einer Tabaksbeutel-Naht bei Magen-, Darm-, Lungen-Operationen oder dergleichen, mit den im Oberbegriff des Anspruches 1 angegebenen Merkmalen.

Zum Legen einer Tabaksbeutel-Naht mit Hilfe der eingangs genannten Nahtklemme z.B. zum Verschließen des Dickdarms bei bestimmten Darmoperationen, wie Darm-Teilentfernungen, ist bisher ein üblicher, relativ langer Operationsschnitt in der Bauchdecke notwendig.Bisher konnte nämlich eine derartige Naht aufgrund der scherenartigen Ausbildung der Nahtklemme nicht im Rahmen der sogenannten endoskopischen Chirurgie gelegt werden, bei der die notwendigen Beobachtungsinstrumente, wie Endoskope, und chirurgischen Instrumente über die Bauchdecke durchstoßende Trokare eingeführt werden. Insofern waren bestimmte Operationen, bei denen z.B. eine Tabaksbeutel-Naht gelegt werden muß, nicht mit den Mitteln der endoskopischen Chirurgie durchführbar.

Aus der EP-A-0 512 725 ist eine chirurgische Klemme zur Anwendung in der endoskopischen Chirurgie bekannt. Bei dieser Klemme sind im Bereich des in das Körperinnere einführbaren Rohrendes eines Einführungsrohres zwei Klemmenbacken angeordnet, die in Schließstellung parallel zur Rohrlängsrichtung verlaufen und mit ihren Querschnittsabmessungen innerhalb des Rohrumrisses bleiben. Einer der beiden Klemmenbacken ist stationär gelagert, wogegen der zweite Klemmenbacken über eine Getriebevorrichtung vom körperäußeren Ende des Einführungsrohres her zu öffnen ist.

Zwar zeigt die EP-A-0 512 725 eine endoskopisch anwendbare, die Merkmale des Oberbegriffes des Anspruches 1 aufweisende Klemme oder Zange, diese ist jedoch aufgrund der Klemmenbackenausrichtung nicht als Nahtklemme geeignet. Aufgrund der zur Längsachse des Rohres parallelen Längsausrichtung der Klemmenbacken im geschlossenen Zustand ist das Durchführen einer Operationsnadel zum Herstellen einer Naht durch die Klemmenbacken nicht möglich.

Ausgehend von dieser Problematik liegt der Erfindung die Aufgabe zugrunde, eine chirurgische Nahtklemme, und insbesondere Tabaksbeutel-Nahtklemme, der gattungsgemäßen Art so weiterzubilden daß sie im Rahmen der endoskopischen Chirurgie anwendbar ist.

Diese Aufgabe wird durch die im Kennzeichnungsteil des Anspruches 1 angegebenen Merkmale gelöst. Die Nahtklemme ist laut Anspruch 1 als endoskopierbare, durch ein Trokar in das Körperinnere einführbare Nahtklemme mit einem Einführungsrohr ausgebildet, im Bereich dessen in das Körperinnere einführbaren, inneren Rohrendes mindestens ein Klemmenbacken relativ zum zweiten Klemmenbacken schwenkbar angetrieben gelagert ist. Darüber hinaus ist im Bereich des außerhalb des Körpers verbleibenden, äußeren Rohrendes ein Antrieb angeordnet, der über ein Getriebe mit dem mindestens einen schwenkbar angetriebenen Klemmenbacken gekoppelt ist.

Aufgrund dieser für chirurgische Klemmen und Zangen aus der EP-A-0 512 725 bekannten Ausgestaltung ist es grundsätzlich möglich, die Nahtklemme im Rahmen der endoskopischen Chirurgie zu verwenden, wobei es sich versteht, daß das Rohr sowie insbesondere die am inneren Rohrende angeordneten Klemmenbacken zumindest im geschlossenen Zustand so angeordnet und dimensioniert sind, daß diese Teile durch die Innenöffnung des Trokars passen und dieser somit als Schleuse in der Bauchdecke für die Nahtklemme dienen kann.

Laut Kennzeichnungsteil des Anspruches 1 sind die Klemmenbacken der endoskopischen Nahtklemme im geschlossenen Zustand mit ihrer Längsrichtung in einem kleinen spitzen Winkel zur Längsachse des Einführungsrohres angeordnet, wobei der Winkel so bemessen ist, daß die Klemmbacken in Schließstellung innerhalb des Umrisses der Einführungsrohrs verbleiben. Durch diese erfindungsgemäße Schrägstellung wird einerseits erreicht, daß ein in den Klemmenbacken ausgebildeter Nadelkanal von beiden Enden der Klemmenbacken her gut zugänglich ist. Andererseits verbleiben dadurch die Klemmenbacken im geschlossenen Zustand bezogen auf die Längsachse des Einführungsrohres innerhalb dessen Umrisses, so daß die Nahtklemme auch mit den schräggestellten Klemmenbacken durch die Innenöffnung des Trokars geschoben werden kann.

Weitere Merkmale, Einzelheiten und Vorteile der Erfindung sind den Unteransprüchen und der nachfolgenden Beschreibung entnehmbar, in der ein Ausführungsbeispiel des Erfindungsgegenstandes anhand der beiliegenden Figuren näher erläutert wird. Es zeigen:
- Fig. 1: eine Seitenansicht einer erfindungsgemäßen Tabaksbeutel-Nahtklemme,
- Fig. 2: eine Seitenansicht der Klemme aus Pfeilrichtung II gem. Fig. 1 in geschlossenem Zustand der Klemmenbacken und
- Fig. 3: eine teilweise entlang der Schnittlinie III-III nach Fig. 1 geschnittene Seitenansicht der Klemme aus Pfeilrichtung II mit geöffneten Klemmenbacken.

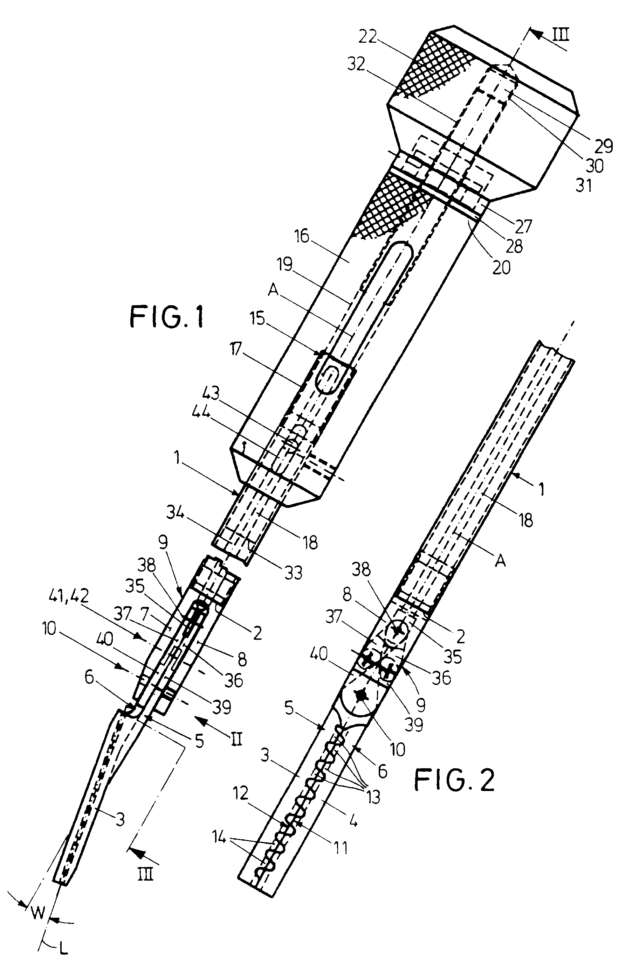

Eine erfindungsgemäße endoskopische Tabaksbeutel-Nahtklemme, wie sie in den Fig. 1 bis 3 dargestellt ist, weist als Hauptbestandteil ein Einführungsrohr 1 auf, das durch ein (nicht dargestelltes) Trokar in der Bauchdecke des Patienten in das Körperinnere einführbar ist. Im Bereich des in das Körperinnere einführbaren, inneren Rohrendes 2 sind zwei Klemmenbacken 3,4 schwenkbar angetrieben gelagert, wobei beide Klemmenbacken 3,4 jeweils als Arm eines doppelarmigen Klemmenhebels 5,6 ausgebildet sind. Letztere sind zwischen den Gabelschenkeln 7,8 eines am inneren Rohrende 2 befestigten, gabelförmigen Lagerkopfes 9 mittels einer gemeinsamen Schwenkachse 10 schwenkbar gelagert.

Die einander zugewandten Klemmflächen 11,12 der beiden Klemmenbacken 3,4 sind in üblicher Weise jeweils wellenförmig ausgestaltet, wobei die Erhebungen 13 in den Klemmflächen 11,12 jeweils durch einen in Fig. 1 und 2 strichliert dargestellten, in Längsrichtung L der Klemmenbacken 3,4 verlaufenden Nadelkanal 14 durchsetzt sind. Durch diese Nadelkanäle 14 wird mit Hilfe von Nadeln im geschlossenen Zustand der Klemme ein Faden in gleichläufiger Richtung zum Legen der Tabaksbeutel-Naht hindurchgezogen.

Die beiden Klemmenbacken 3,4 verlaufen im übrigen in geschlossenem Zustand (Fig. 1,2) mit ihrer Längsrichtung L in einem kleinen spitzen Winkel W zur Längsachse A des Einführungsrohres 1. Dieser Winkel W beträgt etwa 10° und ist so bemessen, daß die Klemmenbacken 3,4 in Schließstellung (Fig. 1,2) innerhalb des kreisförmigen Umrisses des Einführungsrohres 1 bezogen auf eine parallel zur Längsachse A des Rohres 1 verlaufende Betrachtungsrichtung der Klemmbacken 3,4 liegen. Durch diese Schrägstellung sind die Mündungsöffnungen des Nadelkanals 14 bequem zugänglich, was sich bei der Einführung von Nadeln in den Nadelkanal 14 mit Hilfe eines endoskopischen Manipulators besonders günstig auswirkt.

Auf das außerhalb des Körpers verbleibende, äußere Rohrende 15 des Einführungsrohres 1 ist koaxial ein im wesentlichen zylindrisches Griffstück 16 mittels der Schraubverbindung 17 aufgeschraubt, wobei das Griffstück 16 eine koaxial mit der Rohröffnung 18 verlaufende, durchgehende Innenbohrung 19 aufweist. An dem dem Einführungsrohr 1 abgewandten Stirnende 20 des Griffstückes 16 ist eine ebenfalls koaxial verlaufende Ringschulter 21 vorgesehen, auf der ein Spindelrad 22 mit seiner zylindrischen Innenöffnung 23 drehbar gelagert ist. Am äußeren Umfang der Ringschulter 21 ist ferner eine Umfangsnut 24 vorgesehen, in die von außen Madenschrauben 25 als Vorsprünge eingreifen. Diese Madenschrauben sind durch entsprechende Gewindebohrungen 26 in dem Ringkragen 27 um die zylindrische Innenöffnung 23 des Spindelrades 22 hindurchgeschraubt und legen letzteres in längsaxialer Richtung des Einführungsrohres 1 fest. Zwischen der Stirnseite des Ringkragens 27 und dem Griffstück 16 ist ein Gleitring 28 aus Messing eingesetzt.

Das Spindelrad 22 bildet mit seiner koaxial zur Längsachse A des Einführungsrohres 1 angeordneten, von der Innenöffnung 23 ausgehenden Sacklochbohrung 29 mit Innengewinde 30 zusammen mit einem Gewindebolzen 31 in der Innenbohrung 19 des Griffstückes 16 einen Spindeltrieb für die Klemmenbacken 3,4. Dazu steht das Außengewinde 32 des Gewindebolzens 31 mit dem Innengewinde 30 im Spindelrad 22 in Eingriff. Weiterhin ist der Gewindebolzen 31 an seinem dem Spindelrad 22 abgewandten Ende 33 mit einer Schubstange 34 verbunden, die zum gabelförmigen Lagerkopf 9 führt und dort innerhalb der beiden Gabelschenkel 7,8 endet. An diesem entsprechenden Ende 35 der Schubstange 34 sind zwei Kniehebellenker 36, 37 mittels einer gemeinsamen Gelenkachse 38 endseitig angelenkt. Die beiden anderen Enden der Kniehebellenker 36,37 sind jeweils gelenkig mit den beiden Betätigungsarmen 39,40 der Klemmenhebel 5,6 über Gelenkachsen 41,42 verbunden. Aufgrund der vorstehend beschriebenen Konstruktion bilden der Gewindebolzen 31 gemeinsam mit der Schubstange 34 und den beiden Kniehebellenkern 36,37 ein Getriebe zur Übertragung der vom Spindelrad 22 herrührenden Drehantriebsbewegung auf die beiden Klemmenhebel 5,6. Dabei ist ferner die Einheit aus Gewindebolzen 31 und Schubstange 34 in Drehrichtung arretiert. Für diese Dreharretierung ist der Gewindebolzen 31 auf seiner Außenseite mit einer radial abstehenden Erhebung in Form eines Schraubenkopfes 43 einer radial in den Gewindebolzen eingeschraubten Schraube versehen, welcher Schraubenkopf 43 in ein parallel zur Längsachse A des Einführungsrohres 1 darin verlaufendes Langloch 44 eingreift.

Das Öffnen und Schließen der Klemmenbacken 3,4 geschieht wie folgt:

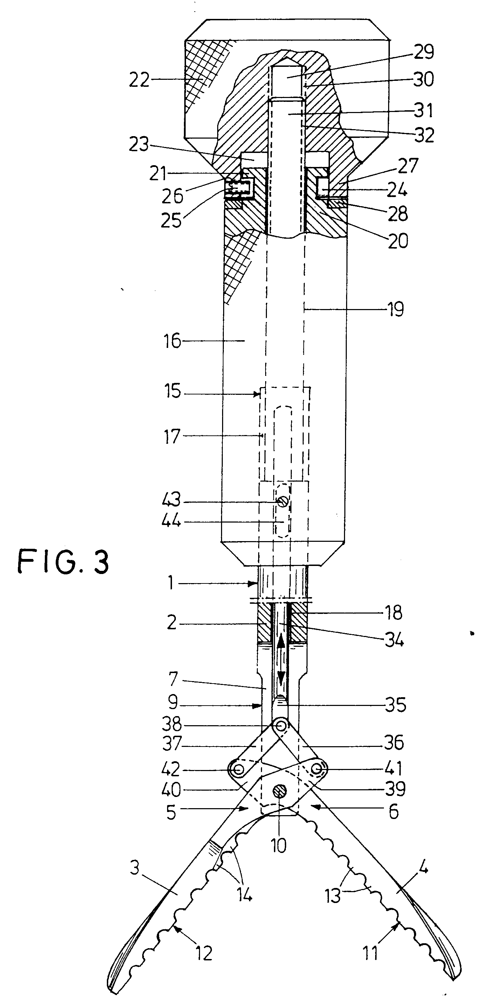

Ausgehend von der in den Fig. 1 und 2 gezeigten Schließstellung der beiden Klemmenbacken 3,4, in der die Kniehebellenker 36,37 und die Betätigungsarme 39,40 innerhalb der beiden Gabelschenkel 7,8 des gabelförmigen Lagerkopfes 9 liegen, wird das Spindelrad 22 drehbetätigt. Durch die Dreharretierung von Gewindebolzen 31 und Schubstange 34 wird die Drehbewegung des Spindelrades 22 nach Art eines Spindeltriebes in eine Schubbewegung von Gewindebolzen 31 und Schubstange 34 in Richtung zum inneren Rohrende 2 des Einführungsrohres 1 umgesetzt. Aufgrund dieser Schubbewegung und der Annäherung der Gelenkachse 38 an die Schwenkachse 10 der beiden Klemmenhebel 5,6 spreizen sich die beiden Kniehebellenker 36,37 nach außen auf und spreizen demzufolge die beiden Betätigungsarme 39,40 der Klemmenhebel 5,6 (Fig. 3). Die Klemmenbacken 3,4 werden damit in ihre Öffnungsstellung übergeführt.

Zum Schließen der Klemmenbacken 3,4 wird das Spindelrad 22 in entgegengesetzter Drehrichtung betätigt, wodurch der Gewindebolzen 31 mit der Schubstange 34 in die entgegengesetzte Richtung koaxial zur Längsachse A verschoben und damit die beiden Klemmenhebel 5,6 über die Kniehebellenker 36,37 wieder eingezogen werden.

## Patentansprüche

1. Chirurgische Nahtklemme, insbesondere Tabaksbeutel-Nahtklemme zum Legen einer Tabaksbeutel-Naht bei Magen-, Darm-, Lungenoperationen oder dergleichen, mit zwei zangenartig relativ zueinander beweglich gelagerten Klemmenbacken (3,4), wobei die Nahtklemme als endoskopierbare, durch ein Trokar in das Körperinnere einführbare Nahtklemme mit einem Einführungsrohr (1) ausgebildet ist, im Bereich dessen in das Körperinnere einführbaren, inneren Rohrendes (2) mindestens ein Klemmenbacken (3) relativ zum zweiten Klemmenbacken (4) schwenkbar angetrieben gelagert ist und wobei im Bereich des außerhalb des Körpers verbleibenden, äußeren Rohrendes (15) ein Antrieb (22) angeordnet ist, der über ein Getriebe (31,34) mit dem mindestens einen schwenkbar angetriebenen Klemmenbacken (3,4) zum Öffnen und Schließen der Nahtklemme gekoppelt ist, **dadurch gekennzeichnet, daß** die Klemmenbacken (3,4) im geschlossenen Zustand mit ihrer Längsrichtung (L) in einem kleinen spitzen Winkel (W) zur Längsachse (A) des Einführungsrohres (1) verlaufen, und wobei der Winkel so bemessen ist, daß die Klemmbacken (3,4) in Schließstellung innerhalb des Umrisses des Einführungsrohres verbleiben.

2. Nahtklemme nach Anspruch 1, **dadurch gekennzeichnet, daß** Antrieb und Getriebe als Spindeltrieb mit einem längsaxial bezüglich der Rohrachse (A) festgelegten, koaxial dazu drehbar gelagerten Spindelrad (22) und einer in Drehrichtung arretierten, längsaxial im Einführungsrohr (1) verschiebbaren Schubstange (34,31) ausgebildet sind, die mit einem Außengewinde (32) an ihrem äußeren Ende mit einem Innengewinde (30) des Spindelrades (22) in Eingriff steht und die mit ihrem inneren Ende (35) mit dem mindestens einen schwenkbar gelagerten Klemmenbacken (3,4) gelenkig gekoppelt ist.

3. Nahtklemme nach Anspruch 2, **dadurch gekennzeichnet, daß** beide Klemmenbacken (3,4) jeweils als Arm doppelarmiger Klemmenhebel (5,6) ausgebildet sind, die um eine Achse (10) am inneren Rohrende (2) schwenkbar gelagert sind und deren Betätigungsarme (39,40) jeweils über Kniehebellenker (36,37) gelenkig mit dem inneren Ende (35) der Schubstange (34) verbunden sind.

4. Nahtklemme nach Anspruch 3, **dadurch gekennzeichnet, daß** am inneren Rohrende (2) ein gabelförmiger Lagerkopf (9) vorgesehen ist, zwischen dessen Gabelschenkel (7,8) die Klemmenhebel (5,6) um eine gemeinsame Achse (10) schwenkbar gelagert sind.

5. Nahtklemme nach Anspruch 4, **dadurch gekennzeichnet, daß** das innere Ende (35) der Schubstange (34) und die Kniehebellenker (36,37) zumindest bei geschlossenen Klemmenbacken (3,4) zwischen den Gabelschenkels (7,8) des gabelförmigen Lägerkopfes (9) angeordnet sind.

6. Nahtklemme nach einem der Ansprüche 2 bis 5, **dadurch gekennzeichnet, daß** am äußeren Rohrende (15) ein im wesentlichen zylindrisches Griffstück (16) angeordnet ist.

7. Nahtklemme nach Anspruch 6, **dadurch gekennzeichnet, daß** das Spindelrad (22) am Griffstück (16) drehbar gelagert und mittels in eine ringförmige Umfangsnut (24) am Griffstück (16) eingreifender Vorsprünge (25) am Spindelrad (22) längsaxial festgelegt ist.

8. Nahtklemme nach einem der Ansprüche 2 bis 7, **dadurch gekennzeichnet, daß** zur Dreharretierung der Schubstange (34,31) diese mit einer radial abstehenden Erhebung (43) versehen ist, die in ein parallel zur Längsachse (A) des Einführungsrohres (1) verlaufendes Langloch (44) eingreift.

## Claims

1. A surgical suture clamp, in particular a purse string suture clamp for applying a purse string suture during the performance of gastric, intestinal and pulmonary operations or the like, with two clamp jaws (3, 4) movably positioned one in relation to the other in a forceps-like manner, the suture clamp being structured as an endoscopable suture clamp, to be led into the body via a trocar, with a leading-in tube (1), in the vicinity of the inner tube end (2) of which, to be led into the body, at least one clamp jaw (3) is positioned to be pivotably driven in relation to the second clamp jaw (4), and a drive (22) being arranged in the vicinity of the outer tube end (15) remaining outside the body and is coupled, by way of a transmission (31, 34), with the at least one pivotably driven clamp jaw (3, 4) for the opening and the closing of the suture clamp, **characterized in that** in the closed condition, the clamp jaws (3, 4) in their longitudinal direction (L) extend at a small acute angle (W) to the longitudinal axis of the leading-in tube (1), and the angle being dimensioned such that the clamp jaws (3, 4), in their closed position, remain within the contour of the leading-in tube.

2. A suture clamp according to claim 1, **characterized in that** the drive and the transmission are formed as a spindle drive with a spindle wheel (22) arrested longitudinally axially in relation to the tube axis (A) and positioned coaxially rotatably thereto and a push bar (34, 31) arrested in the direction of rotation and longitudinally axially displaceable in the leading-in tube (1), which push bar (34, 31), with an external thread (32) at its outer end, is in engagement with an internal thread (30) of the spindle wheel (22) and which, with its inner end (35), is coupled for articulation with the at least one pivotably positioned clamp jaw (3, 4).

3. A suture clamp according to claim 2, **characterized in that** the clamp jaws (3, 4) are each formed as an arm of double-armed clamp levers (5, 6), which are positioned at the inner tube end (2) pivotably about an axis (10) and of which the operating aims (39, 40) are connected by way of articulated lever bars (36, 37) for articulation with the inner end (35) of the push bar (34).

4. A suture clamp according to claim 3, **characterized in that** a forked bearing head (9) is provided at the inner tube end (2), between the fork legs (7, 8) of which forked bearing head (9) the clamp levers (5, 6) are positioned pivotably about a common axis (10).

5. A suture clamp according to claim 4, **characterized in that** at least with the clamp jaws (3, 4) closed, the inner end (35) of the push bar (34) and the articulated lever bars (36, 37) are arranged between the fork legs (7, 8) of the forked bearing head (9).

6. A suture clamp according to one of claims 2 to 5, **characterized in that** a substantially cylindrical handle member (16) is arranged at the outer tube end (15).

7. A suture clamp according to claim 6, **characterized in that** the spindle wheel (22) is pivotably supported on the handle member (16) and is longitudinally axially arrested by means of projections (26) on the spindle wheel (22) engaging with an annular circumferential groove on the handle member (16).

8. A suture clamp according to one of claims 2 to 7, **characterized in that** for arrest of rotation, the push bar (34, 31) is provided with a radially projecting elevation (43), which engages with an oblong hole (44) extending parallel to the longitudinal axis (A) of the leading-in tube (1).

## Revendications

1. Pince chirurgicale à suture, notamment pince à suture en bourse pour appliquer une suture en bourse lors d'opérations de l'estomac, de l'intestin, des poumons ou analogues, comportant deux mâchoires (3,4), qui sont montées de manière à être déplaçables l'une par rapport à l'autre à la manière de pinces, dans laquelle la pince à suture est agencée sous la forme d'une pince à suture pouvant être insérée dans un endoscope et pouvant être introduite par un trocart à l'intérieur du corps et comportant un tube d'introduction (1), au moins une mâchoire (3) de la pince étant montée en étant entraînée de manière à pouvoir pivoter par rapport à la seconde mâchoire de la pince, au voisinage de l'extrémité intérieure (2) du tube, qui peut être introduite à l'intérieur du corps, et dans laquelle dans la zone de l'extrémité extérieure (15) du tube, qui subsiste à l'extérieur du corps, est disposé un dispositif d'entraînement (22) qui est accouplé par l'intermédiaire d'un mécanisme (31,34) à la au moins une mâchoire (3,4) de la pince, qui est entraînée de manière à pouvoir pivoter, pour ouvrir et fermer la pince à suture, caractérisé en ce qu'à l'état fermé, la direction longitudinale (L) des mâchoires (3,4) de la pince fait un petit angle aigu (W) par rapport à l'axe longitudinal (A) du tube d'introduction (1), et dans lequel l'angle est dimensionné de telle sorte que dans la position fermée, les mâchoires (3,4) de la pince restent en-deçà du tube d'introduction

2. Pince à suture selon la revendication 1, caractérisé en ce que le dispositif d'entraînement et le mécanisme sont agencés sous la forme d'un dispositif d'entraînement à broche comportant un écrou de broche (22), fixé dans la direction de l'axe longitudinal par rapport à l'axe (A) du tube et monté de manière à pouvoir tourner coaxialement à cet axe, et une tige de poussée (34,31), qui est bloquée en rotation et est translatable dans la direction de l'axe longitudinal à l'intérieur du tube d'introduction (1) et qui engrène par un filetage extérieur (32) situé sut son extrémité extérieure, avec un taraudage (30) de l'écrou de broche (22) et qui est accouplé de manière articulée, par son extrémité intérieure (35), à la au moins une mâchoire (3,15) de la pince, montée de manière à pouvoir pivoter.

3. Pince à suture selon la revendication 2, caractérisée en ce que les deux mâchoires (3,4) de la pince sont agencées chacune sous la forme d'un bras de leviers (5,6) de la pince à deux bras, qui sont montés de manière à pouvoir pivoter autour d'un axe (10) sur l'extrémité intérieure (2) du tube et dont les bras d'actionnement (39,40) sont reliés d'une manière articulée, respectivement par l'intermédiaire de leviers oscillants à genouillère (36,37), à l'extrémité intérieure (35) de la tige de poussée (34).

4. Pince à suture selon la revendication 2, caractérisée en ce que sur l'extrémité intérieure (2) du tube est prévue une tête de support en forme de fourche (9), entre les branches (7,8) de laquelle les leviers (5, 6) de la pince sont montés de manière à pouvoir pivoter autour d'un axe commun (10).

5. Pince à suture selon la revendication 4, caractérisée en ce que l'extrémité intérieure (35) de la tige de poussée (34) et les leviers oscillants à genouillère (36,37) sont disposés, au moins lorsque les mâchoires de serrage (3,4) sont fermées, entre les branches (7,8) de la fourche de la tête de support (9) en forme de fourche.

6. Pince à suture selon l'une des revendications 2 à 5, caractérisée en ce qu'un élément de préhension essentiellement cylindrique (16) est disposé sur l'extrémité extérieure (15) du tube.

7. Pince à suture selon la revendication 6, caractérisée en ce que l'écrou de broche (22) est monté de manière à pouvoir tourner sur l'organe de préhension (16) et est bloqué dans la direction de l'axe longitudinal sur l'écrou de broche (22) au moyen d'appendices saillants (25), qui s'engagent dans une gorge annulaire circonférentielle (24) formée dans l'organe de préhension (16).

8. Pince à suture selon l'une des revendications 2 à 7, caractérisée en ce que pour le blocage en rotation de la tige de poussée (34,31), cette dernière est pourvue d'on bossage radial (43) qui s'engage dans un trou allongé (44) qui s'étend parallèlement à l'axe longitudinal (A) du tube d'introduction (1).
